# EUROPEAN PATENT APPLICATION

(11) **EP 2 438 870 A1**
(43) Date of publication of application: **11.04.2012**
(21) Application number: 10013397.4
(22) Date of filing: 07.10.2010
(51) Int. Cl.: A61B 17/80

(54) **Orthopedic implant device and system**

(71) Applicant: Instrumentaria d.d., 10360 Sesvete (HR)
(72) Inventor: Dizdar, Adnan, Prim. M. Sc., 71000 Sarajevo (BA)
(74) Representative: Clarenbach, Carl-Philipp

(57) **Abstract**

The present invention relates to an orthopedic implant device (2) for fixation of a fractured bone (5), in particular of the human body, comprising at least one opening (9) extending from an upper surface (7) to a lower surface (8), whereby the at least one opening (9) includes a first hole-portion (11) and a second hole-portion (12), the two hole-portions (11,12) overlapping each other at their ends to form a combination-hole (10) with the first hole-portion (11) being shaped as first elongated hole-portion (11), both hole-portions (11,12) being designed to receive a bone-screw (3,4) at a selectable angle at the same time. It is proposed that the second hole-portion (12) is shaped as second elongated hole-portion (12) and that the longitudinal axis (13) of the first elongated hole-portion (11) is arranged at an angle (α) to the longitudinal axis (14) of the second elongated hole-portion (12).

## Description

The present invention relates to an orthopedic implant device for fixation of a fractured bone, in particular of the human body that comprises at least one opening extending from an upper surface to a lower surface, into which at least one screw can be placed. Standard types of orthopedic implant devices contain standard circular or elongated holes which allow one single screw to be placed within at a certain angle with respect to the implant device. The opening of recent implant devices includes a first hole-portion and a second hole-portion to receive two bone-screws at the same time, the two hole-portions overlapping each other at their ends to form a combination-hole, with at least the first hole-portion being designed elongated.

The present invention also relates to an orthopedic implant system, in particular for fixation of a fractured bone, in particular for the human body, including at least one orthopedic implant device that comprises a combination-hole and at least one bone-screw to be placed in the combination-hole.

Applying one screw to the known implant devices provides sufficient dynamic and inter-fragmentary firmness. Providing two hole-portions forming the combination-hole allows the surgeon to decide whether to place one or two screws into the opening. Applying the second screw into the remaining hole-portion of the combination-hole ensures not just increased firmness of one screw, but also an additional axial and rotational stability of the implant device.

A known orthopedic implant device according to the preamble of claim 1 is disclosed in the international patent application WO 2009/140742 A1. This implant device comprises a combination-hole that includes a circular-shaped hole-portion and an elongated hole-portion that overlap each other at their ends forming the one opening. In this regard it shall be noted that by the term "overlapping at their ends" an arrangement of the hole-portion is meant such that one hole-portion does not cross the other hole-portion, but that the two hole-portions have a common beginning or end. For the known implant device this means for example that the elongated hole-portion ends in the circular-shaped hole-portion. An elongated hole or hole portion is characterized in comprising a longitudinal axis along which the opening extends and two opposite end sections that preferably each comprise a radius the centre of which defines the end of the respective longitudinal axis. An elongated hole or hole-portion can also be shaped oval such that the two opposite longitudinal sides that substantially extend parallel to the longitudinal axis are at least partially curved. Each hole-portion of the known combination-hole is designed to receive a bone-screw at a selectable angle with respect to the implant device. Providing two screws at selectable angles through one opening of the implant device allows capturing peripheral segments of the bone structure and provides a firm attachment of the implant device to the bone while the two screws are arranged within a small area of the implant device or the bone to be fixed. Due to the shape of the known combination-hole with a first elongated hole-portion and a second circular hole-portion it is necessary to use screws with a reduced head size to allow two screws to be placed within the combination-hole at the same time.

### Object of the invention

It is the object of the present invention to provide an implant device which allows for a higher stability and firmness of the fixation of the fractured bone and of the implant device itself and which allows regular bone-screws with regular head-sizes to be placed within the combination-hole at the same time.

### Summary of the invention

The inventive orthopedic implant device has the advantage of providing a firmer and stiffer fixation of the bones due to a more variable placement of the screws and of being highly resilient. The inventive implant device is characterized in that the second hole-portion is shaped as second elongated hole or second elongated hole-portion and that the longitudinal axis of the first elongated hole or first elongated hole-portion is arranged at an angle to the longitudinal axis of the second elongated hole or hole-portion. The arrangement of the longitudinal axis of the two elongated holes or hole-portions provides a combination-hole having a heart-shaped or drusy-shaped contour. In other words, the inventive combination-hole is shaped heart- or drusy-like. Both elongated holes or hole-portions are designed such that the screw placed into the respective hole-portion can be angled in different or selectable directions. During operation the surgeon can freely decide in which angle the respective screw is to be drilled into the bone. Due to the heartshape of the combination-hole the screws can be fixed such that a very strong and durable connection is obtained and that more fractured parts of the bone, even bone parts which are not directly beneath the bone plate can be reached and fastened/fixed by the implant device. The angled arrangement of the longitudinal axes allows the screws to be placed close to one another while acting onto the bone plate and/or the bone in different directions and in particular in opposite directions to one another. The advantageous shape of the combination-hole also allows two regular bone-screws with regular head-sizes to be placed within the combination-hole at the same time. The heads can be arranged such that the position of the first screw does not conflict the position of the second screw. According to a preferred embodiment of the invention the screws can be placed in the combination-hole such that their axes define a common plane. With the preferred combination-hole an implant device is provided that requires less holes than implant devices of the prior art while providing the same or higher stability and firmness of the fixation of the fractured bone.

According to a preferred embodiment of the invention each hole-portion is designed threadless. The threadless design of the respective hole-portion allows the screws to be placed at more variable angles and provides a less cost-intensive implant device. Of course, according to an alternative embodiment of the invention, at least one, preferably both hole-portions are provided with a respective thread for bone-screws with threaded screw heads.

Preferably, each hole-portion comprises a diminution in its axial direction. The axial direction is to be seen from the upper surface to the lower surface of the implant device. Thus, in the axial direction of the respective hole-portion the diminution reduces the available opening size of the respective hole-portion. The diminution is preferably selected such that its inner diameter or distance defining the opening is substantially equal to the diameter of the bolt of the screw to be placed within the respective hole-portion. The diminution of the respective hole-portion defines the radial position of the respective screw, while allowing the screw to be angled within the respective hole-portion.

According to a preferred embodiment of the invention the upper section between the diminution and the upper surface of the implant device forms a bearing surface for a head of the respective screw. The bearing surface is particularly designed such that a large contact area is provided for a firm and stable connection.

The bearing surface is preferably designed at least partially spherical or cone-shaped, in particular depending on the shape of the screw head. While the cone-shaped upper section between the diminution and the upper surface of the implant device can be realized easier and less cost-intensive, the spherical design allows for a better adjustment of the axis of the respective screw with regard to its angled alignment. The lower section between the diminution and the lower surface of the implant device can be cone-shaped or spherical.

According to a preferred embodiment of the invention the inner width of the combination-hole in the overlapping area is larger, in particular slightly larger than the inner width of the respective elongated hole-portion. In the overlapping area, where the respective-hole-portions overlap each other, the largest inner width of the combination-hole is provided. Due to the larger inner width in the overlapping area it is possible to place a bone-screw that comprises a larger diameter than regular bone-screws in the middle of the combination-hole. Such kinds of bone-screws are for example cancellous or cortical bone-screws. The preferred design of the combination-hole therefore allows the surgeon to place different screws within the combination-hole either in one of the elongated hole-portions or within the centre of the combination-hole. Due to the enlarged inner width of the combination-hole in the overlapping area a bone-screw that can be placed within the elongated hole-portions at a selectable angle within a certain angular range can be placed within the centre or the overlapping area of the combination-hole at an angle within an increased angular range.

The inner width of the respective elongated hole-portion preferably increases along the respective longitudinal axis towards the overlapping area. In this way a smooth merging of the hole-portions in the overlapping area is provided. The inner width of the hole-portions increases towards the overlapping area preferably slowly or slightly.

According to a preferred embodiment of the invention the lower surface is adapted to be attached to a bone. In particular, the lower surface is designed such that in a cross-sectional view the lower surface is formed arcuated or curved. In this way, the implant device can be closely attached to the bone. The upper surface of the implant device is preferably designed to extend parallel to the lower surface.

According to a preferred embodiment of the invention the orthopedic implant device is designed as a bone plate. The bone plate extends into one direction essentially parallel to the axis of the bone. Along its longitudinal axis the bone plate can be shaped straight or bent, depending on the future location on the bone where the bone plate is to be attached. The bone plate is preferably made of a material that is bio-compatible, such as plastic, titanium or steel or a combination thereof.

Furthermore, it is preferably provided that the bone plate comprises at least two sections, the sections being arranged at an angle to one another. The bone plate can be provided with only one or two or more than two combination-holes as described above. Also, the bone plate can be provided with one or more conventional circular or elongated holes additionally.

According to a further preferred embodiment the combination-hole comprises a diminution between the two elongated hole-portions. Instead of being merged directly at their ends, the centres of the respective ends of the elongated holes can be arranged apart from another, thereby forming the diminution between the two elongated hole-portions. By doing so, if a screw is placed near to the merging area a large contact area is provided by the bearing surface of the upper section of the opening. The diminution is preferably designed such that it prevents a screw from moving from one hole-portion to the other hole-portion.

The inventive orthopedic implant system is characterized in that the implant device is designed according to one of the embodiments described above. With the inventive implant system a surgeon can decide during operation how many screws and in what angle the respective screw is to be drilled into the bone through a hole-portion of the combination-hole. By providing two screws, one in each hole-portion of the combination-hole, a firm and stable connection between the implant device, in particular the bone plate and the bone can be realized within a small area, due to the advantageous heart- or drusy-shaped design of the combination-hole.

In the following detailed description the invention shall be explained in conjunction with the accompanying drawings in which:
- Figure 1: is a schematic drawing of an orthopedic implant system;
- Figure 2: is a schematic top view of an inventive bone plate comprising combination-holes;
- Figure 3: is a top view of the bone plate comprising alternatively designed combination-holes;
- Figure 4: is a top view of the bone plate comprising another alternatively designed combination-hole;
- Figure 5: is a cross-sectional view of the bone plate and
- Figure 6: is an exemplary embodiment of an inventive bone plate in a perspective view.

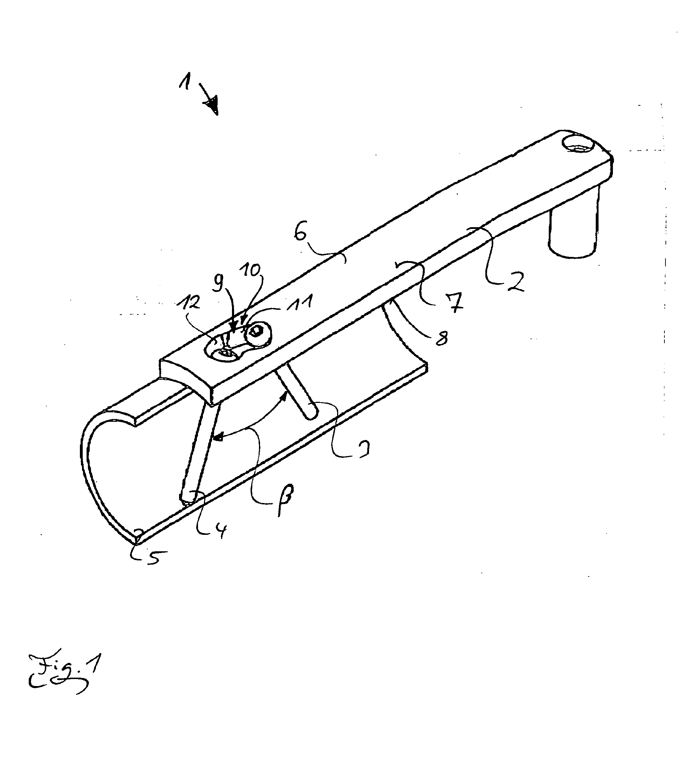

Figure 1 shows an orthopedic implant system 1 that comprises an orthopedic implant device 2 and two bone-screws 3, 4 that are used to attach the implant device 2 to the bone 5 of the human body, which is drawn schematically in Figure 1. The implant device 2 is designed as bone plate 6 including an upper surface 7 and a lower surface 8. The lower surface 8 is adapted to be attached or to fit onto the curvature of the bone 5. For this the lower surface 8 comprises an arcuated cross section with a radius preferably equivalent to the radius of the bone. Through the bone plate 6 an opening 9 extends from the upper surface 7 to the lower surface 8. The opening 9 is designed as combination-hole 10 having a first elongated hole-portion 11 and a second elongated hole-portion 12, the two elongated hole-portions 11, 12 overlapping at their ends. Into each hole-portion 11, 12 one of the two bone-screws 3, 4 is placed.

The combination-hole 10 is designed such that the bone-screws 3, 4 can be placed into the respective hole-portion 11, 12 at a selectable angle, for example as shown in Figure 1 such that the bolts of the bone screws 3, 4 point into different, in particular opposite directions.

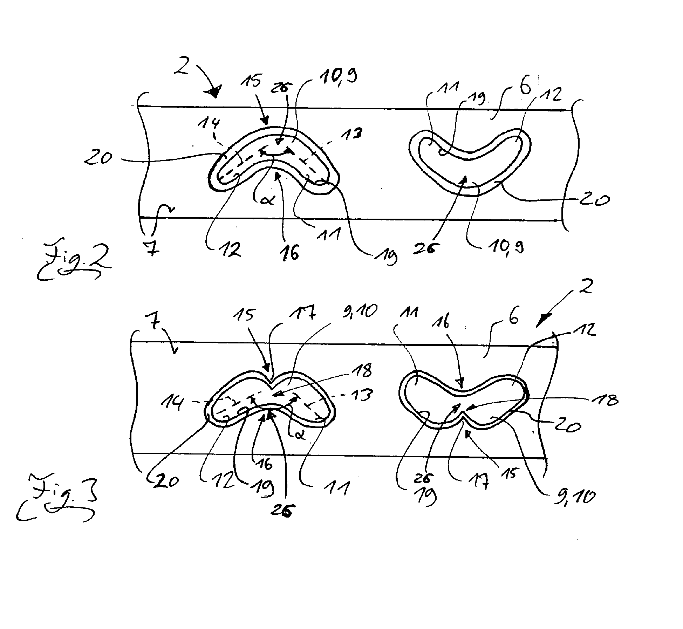

With reference to Figures 2 to 5 the design of the combination-hole 10 shall be explained in more detail.

Figure 2 is a top view onto a preferred embodiment of the bone plate 6. According to this exemplary embodiment the bone plate 6 comprises at least two of the combination holes 10 that are arranged along the longitudinal axis of the bone plate 6 in an alternating arrangement with respect to their own orientation. It can be seen from Figure 2 that the two longitudinal hole-portions 11, 12 merge or overlap at their ends forming a heart-shaped or drusy-shaped opening 9. The elongated hole-portions 11, 12 both comprise longitudinal axis 13, 14 that are arranged at an angle α to one another. The overlapping area 26 of the two longitudinal hole-portions 11, 12 is designed such that the hole-portions 11, 12 merge into one another smoothly. The heart-shaped combination-hole 10 forms a peak 15 on a first side and a dimple 16 at the opposite side. According to a preferred embodiment of the invention the centre of the end-radius of one longitudinal hole-portion 11 is also the centre for the end-radius of the second longitudinal hole-portion 12, such that the centre is right in the middle of the combination-hole 10 and equals to the intersection point of the longitudinal axes 13 and 14. According to an alternative embodiment of the invention the respective centres of the end-radius can be arranged spaced apart from another or in an overlapping manner.

It can be seen in Figure 2 that the elongated hole-portions 11 and 12 comprise curved side lines/walls such that each elongated hole-portion 11 and 12 is at least partially shaped oval.

Figure 3 is a top view onto the bone plate 6 that comprises combination-holes 10 according to an alternative embodiment. The combination-holes 10 of Figure 3 differ from the previous described combination-holes 10 in that the centre of the end-radius of the respective longitudinal hole-portion 11, 12 are spaced apart from another, whereby the area between the longitudinal hole-portions 11, 12 is not merged smoothly, but formed corresponding to the actual design of the respective elongated hole-portion 11, 12. According to the spaced apart arrangement as described above, the peak 15 includes a dent 17. The dent 17 forms a diminution 18 between the two longitudinal hole-portions 11, 12. The diminution 18 or the dent 17 have the advantage that if one of the screws 3, 4 is placed near to the middle of the combination hole 10, an enlarged contact area is provided onto which a head of the respective screw 3, 4 can act. Alternatively, the dent can be designed as flattened peak.

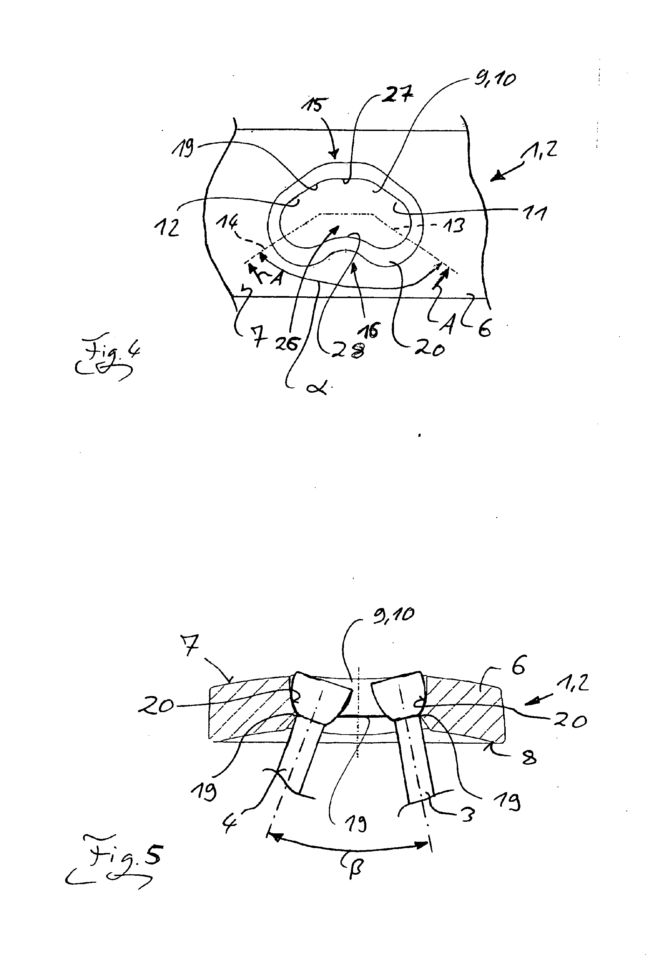

Another alternative embodiment of the implant device 2 is illustrated in Figure 4. According to this embodiment the respective combination-hole 10 particularly differs from the above-described embodiments such that the inner width of the overlapping area 26 is larger than the inner width of the elongated hole-portions 11 and 12. Furthermore, the inner width of the respective hole-portion 11, 12 increases slightly along the respective longitudinal axes towards the overlapping area 26 such that a smooth merging of the hole-portions 11, 12 is provided. The peak 15 of the heart-shaped combination-hole 10 comprises a flattened section 27. The dimple 16 preferably provides - as shown in Figure 4 - a curved section 28 opposite to the flattened section 27. The design disclosed in Figure 4 allows the placement of a bone-screw in the centre or in the overlapping area 26 of the combination-hole 10. In particular, it allows for the placement of a bone-screw that comprises a larger diameter than the bone-screws to be placed within the respective elongated hole-portion 11, 12. Preferably, a so-called cancellous or cortical bone-screw can be placed within the overlapping area 26 of the combination-hole 10. Thus, during operation, the surgeon cannot only decide whether he wants to place one or two screws within the combination-hole 10, he can also choose to place a certain type of bone-screw within the overlapping area 26 of the combination-hole 10 at a vertical or slant arrangement with regard to the bone plate 6.

Figure 5 shows a cross-sectional view of the bone plate 6 along the line A-A through the combination-hole 10 as shown in Figure 4. In Figure 5 it can be seen that the combination-hole 10 comprises a diminution 19 in its axial direction. The upper section 20 of the combination hole 10 between the diminution 19 and the upper surface 7 is partially designed spherical, while the lower section 21 between the diminution 19 and the lower surface 8 is designed conical. The diminution 19 allows the screws 3, 4 to be angled at a desired angle with respect to the bone plate 6 or the bone 5. During operation a surgeon can select the appropriate angle to the fractured bone. The spherical upper section 20 forms a bearing surface for the head of a screw 3 or 4 and is best used if the head of the respective screw 3, 4 is formed correspondingly, in particular spherical. The spherical design allows for a large contact area between the screw head and the bone plate 6, so that high forces can be transmitted and a firm and safe attachment of the bone plate 6 to the bone 5 can be ensured. According to an alternative embodiment, the upper section 20 or the bearing surface is only partially designed spherical.

According to a further alternative embodiment of the bone plate 6 the upper section 20 is designed at least partially conical. In that case the screws 3, 4 should also be provided with conical-shaped heads.

The above-described embodiments of the invention allow the bone plate 6 to be fixed onto the bone 5 by placing a screw 3, 4 in each longitudinal hole-portion 11, 12, respectively, and drilling it into the bone 5. The second screw 4, 3 placed into the same combination-hole 10 ensures not just an increased firmness of the first screw 3, 4, but also an additional axial and rotational stability of the bone plate 6 with respect to the bone 5. Due to the selectable angles at which the screws 3, 4 can be placed into the respective longitudinal hole-portion 11, 12 it is possible to place the tip of the screws not only directly beneath the bone plate 6, but also in an area aside of the bone plate 6. This is particularly beneficial with respect to diaphysis as it captures peripheral bone fragments as well. Due to the angled arrangement of the longitudinal axes 13 and 14 of the longitudinal hole-portions 11 and 12 a particular firm and stiff attachment of the bone plate 6 to the bone 6 is realized, since the screws 3, 4 can be placed close to one another within a small area of the bone plate 6, while pointing into different directions, in particular to form a truss- like arrangement.

By providing the inventive combination hole 10 in the bone plate 6 the firmness of the bone plate 6 substantially remains. Due to the longitudinal hole-portions 11, 12 the respective screw 3, 4 can be placed in different positions within the respective longitudinal hole 11 12 for a versatile use of the combination-hole 10. With the inventive implant device 2 less screws are required for the fixation of a fractured bone, thus reducing damages to the bone structure, nutritional blood flow and periosteum. Furthermore, if less screws are needed, a smaller insection is required for implanting the device 2 into human body. Also, because of the advantageous design of the combination-hole 10, it is possible to place two regular bone-screws within the combination-hole 10, without the need of providing screwheads with reduced sizes.

The implant device 2 as described above can be used in the following ways: Either a single screw or two screws can be placed within one combination-hole 10. Each screw can be placed such that its axis is at least substantially perpendicular to the extension of the bone plate 6. At least one screw 3, 4 can be placed at a slant angle to the bone plate or the other screw 4, 3. Preferably, the two screws 3, 4 are placed divergent or transversely to one another and/or to the implant device 2.

The implant device 2 as described above provides for a high compression and dynamism between the implant device 2 and the fractured bone 5. It is technically safer and stronger to known implant devices in terms of stability and firmness. It enables a specific manner of insertion of two screws 3, 4 in different positions through the single opening 9, hence enabling greater firmness of both the implant device 2 and the bone 5. The divergent placement of the screws 3, 4 reduces the number of screws needed, in particular in case of osteosynthesis. Since less screws are required, less holes are needed, so that the strength of the bone plate 6 is increased and/or the overall size of the implant device 2, in particular of the bone plate 6, can be reduced. A reduced size of the implant device 2 follows the principals of the minimum invasive technique for surgical incisions.

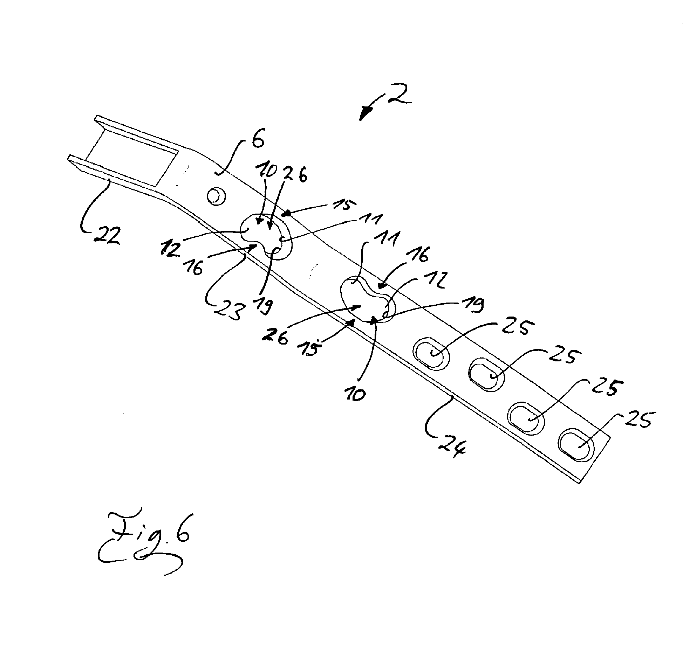

Figure 6 shows an exemplary embodiment of the implant device 2 designed as bone-plate 6. The bone-plate 6 according to this embodiment comprises three sections 22, 23 and 24 that are arranged at certain angles to one another. The middle section 23 is provided with one of the combination holes 10 that substantially equals to the combination-hole 10 disclosed in Figure 4. As mentioned before, the combination-holes 10 are arranged along the longitudinal axis of the bone plate 6 in an alternating manner so that the peak 15 and the dimple 16 are arranged switched or mirrored with respect to the longitudinal axes of the bone plate. The section 24 comprises a second combination hole 10 that is arranged in a mirrored orientation to the combination-hole 10 in section 23. It is obvious that even more combination holes 10 as described above can be provided in the bone plate 6 in either section 22, 23 and/or 24 as shown in Figure 5.

According to the embodiment of Figure 6 the bone plate 6 is additionally provided with standard longitudinal holes 25. The longitudinal axes of the standard longitudinal holes 25 are arranged parallel to the longitudinal middle axis (not shown) of the bone plate 6, whereby the longitudinal holes 25 are arranged on either side of the longitudinal middle axis of the bone plate 6 in an alternating manner. Of course, it is also possible to provide the bone plate 6 with conventional circular holes instead or in addition to the longitudinal or oval holes 25.

According to another embodiment of the orthopedic implant device 1 a plurality of combination-holes 10 is arranged along the longitudinal axis of the bone plate 6, whereby the combination-holes 10 are arranged in groups such that for example a first group of combination-holes comprises a first distance between one combination-hole to the other, while a second group of combination-holes may comprise the same distance or a second distance between one combination-hole to another, while the first and the second groups of combination holes are spaced apart from another by a different distance. A group may comprise at least one, preferably two or more combination holes. It is also possible to provide conventional holes between combination-holes of one group or between the groups of combination-holes.

It is clear to a person of ordinary skill in the art that the design of the bone plate 6 can differ from the disclosed embodiments without leaving the scope of the present invention.

## Claims

1. Orthopedic implant device (2) for fixation of a fractured bone (5), in particular of the human body, comprising at least one opening (9) extending from an upper surface (7) to a lower surface (8), whereby the at least one opening (9) includes a first hole-portion (11) and a second hole-portion (12), the two hole-portions (11,12) overlapping each other at their ends to form a combination-hole (10) with the first hole-portion (11) being shaped as first elongated hole-portion (11), both hole-portions (11,12) being designed to receive a bone-screw (3,4) at a selectable angle at the same time, **characterized in that** the second hole-portion (12) is shaped as second elongated hole-portion (12) and that the longitudinal axis (13) of the first elongated hole-portion (11) is arranged at an angle (α) to the longitudinal axis (14) of the second elongated hole-portion (12).

2. Implant device according to claim 1, **characterized in that** each hole-portion (11,12) is designed threadless.

3. Implant device according to one of the preceding claims, **characterized in that** each hole-portion (11,12) or the combination-hole (10) comprises a diminution (19) in its axial direction.

4. Implant device according to one of the preceding claims, **characterized in that** the upper section (20) of the respective hole-portion (11,12) between the diminution (19) and the upper surface (7) forms a bearing surface to receive a head of the respective screw (3,4).

5. Implant device according to one of the preceding claims, **characterized in that** the bearing surface is at least partially designed spherical or cone-shaped.

6. Implant device according to one of the preceding claims, **characterized in that** the inner width of the combination-hole (10) in the overlapping area (26) is larger than the inner width of the respective elongated hole-portion (11, 12).

7. Implant device according to one of the preceding claims, **characterized in that** the inner width of the respective elongated hole-portion (11, 12) increases slightly along the respective longitudinal axis (13, 14) towards the overlapping area (26).

8. Implant device according to one of the preceding claims, **characterized in that** the lower surface (8) is adapted to be attached to a bone (5), in particular of the human body.

9. Implant device according to one of the preceding claims, **characterized in** being a bone plate (6).

10. Implant device according to one of the preceding claims, **characterized in that** the combination-hole (10) comprises a diminution (18) between the two elongated hole-portions (11,12).

11. Implant device according to one of the preceding claims, **characterized in that** the bone plate (6) comprises at least two sections (22,23,24), the two sections (22,23,24) being arranged, in particular being bent at an angle (β) to one another.

12. Orthopedic implant system (1), in particular for fixation of a fractured bone (5) of the human body, including at least one orthopedic implant device (2) that comprises an opening (9) and at least one bone-screw (3,4) to be placed in the opening (9), **characterized in that** the implant device (2) is designed according to one or more of the preceding claims.
